# EUROPÄISCHE PATENTSCHRIFT

(11) **EP 0 533 231 B1**
(45) Veröffentlichungstag und Bekanntmachung des Hinweises auf die Patenterteilung: **11.01.1995**
(21) Anmeldenummer: 92202531.7
(22) Anmeldetag: 18.08.1992
(51) Int. Cl.: C01B 3/38, C07C 29/151

(54) **Verfahren zur Erzeugung eines Synthesegases für die Methanolsynthese**
Method for synthesis gas generation used in methanol synthesis
Méthode de production de gaz de synthèse pour la fabrication de méthanol

(30) Priorität: 14.09.1991 DE 4130718
(43) Veröffentlichungstag der Anmeldung: 24.03.1993
(73) Patentinhaber: METALLGESELLSCHAFT Aktiengesellschaft, 60015 Frankfurt (DE)
(72) Erfinder: Supp, Emil, W-6057 Dietzenbach (DE); Morgenroth, Rainer, W-6382 Friedrichsdorf (DE)

(56) Entgegenhaltungen:
- EP-A- 0 067 491
- EP-A- 0 269 297
- EP-A- 0 376 419
- DE-A- 753 753
- US-A- 4 866 211

## Beschreibung

Die Erfindung betrifft ein Verfahren zur Erzeugung eines Synthesegases für die Methanolsynthese und Umsetzen des Synthesegases an einem Katalysator zu einem methanolreichen Produktstrom, wobei man ein methanhaltiges Kohlenwasserstoffgas mit Sauerstoff und Wasserdampf in einem Reformierreaktor katalytisch autotherm bei Temperaturen am Reaktoraustritt von 800 bis 1300°C und einem Druck von 10 bis 100 bar umsetzt und aus dem Reformierreaktor ein rohes Synthesegas abzieht, dessen Komponenten vor allem Wasserstoff, Kohlenmonoxid und Kohlendioxid sind.

Ein solches Verfahren ist in EP-A-0067491 beschrieben. Einzelheiten zum katalytisch autotherm arbeitenden Reformierreaktor finden sich in Ullmanns Encyclopaedia of Industrial Chemistry, 5. Auflage, Band A12, Seiten 202-204. Über die ideale Zusammensetzung eines Methanolsynthesegases wird im selben Band auf den Seiten 173 und 174 informiert.

Der Erfindung liegt die Aufgabe zugrunde, einen möglichst kostengünstigen Weg zur Herstellung des für die Synthese geeigneten Methanolsynthesegases zu finden. Erfindungsgemäß gelingt dies beim eingangs genannten Verfahren dadurch, daß man in den Reformierreaktor zur katalytisch autothermen Umsetzung neben dem Kohlenwasserstoffgas, Sauerstoff und Wasserdampf zusätzlich ein an freiem Wasserstoff reiches Gas aus einer anderen Anlage einleitet, wobei man dem Reformierreaktor pro C-Atom 1,2 bis 2,0 Molekühle H₂O, 0,4 bis 0,8 Molekühle O₂ und 0,2 bis 0,5 Molekühle H₂ zuführt, daß man aus dem Reformierreaktor ein rohes Synthesegas mit einem Methangehalt von höchstens 3 Mol.-% abzieht und daraus mit oder ohne Zugabe von Wasserstoff und ohne Entfernung von Kohlendioxid ein für die Methanolsynthese geeignetes Synthesegas erzeugt, in welchem die Konzentrationen der Komponenten H₂, CO und CO₂ ein als Stöchiometriezahl bezeichnetes molares Verhältnis (H₂-CO₂):(CO+CO₂) von 1,97 bis 2,2 ergeben.

Zur Berechnung der Stöchiometriezahl sind in die vorstehend genannte Gleichung die molaren Konzentrationen von Wasserstoff, Kohlenmonoxid und Kohlendioxid im Synthesegas einzusetzen, wie dies dem Fachmann bekannt ist.

Wenn eine genügend große Menge an wasserstoffreichem Gas zum Einleiten in den Reformierreaktor zur Verfügung steht, gelingt es, aus dem Reformierreaktor ein rohes Synthesegas abzuziehen, das bereits die passende Stöchiometriezahl aufweist und deshalb ohne weitere Behandlung der Methanolsynthese zugeführt werden kann. Sollte das rohe Synthesegas aber noch nicht die richtige Stöchiometriezahl besitzen, gibt man dem rohen Synthesegas so viel Wasserstoff zu, bis die gewünschte Stöchiometriezahl erreicht ist.

Bei der bekannten Methanolsynthese entsteht ein freien Wasserstoff und Kohlenoxide enthaltendes Restgas, das man aus der Synthese entfernen muß. Es ist zweckmäßig, dieses Restgas mindestens teilweise in den Reformierreaktor zu leiten. Andererseits kann man das Restgas aufarbeiten und daraus Wasserstoff teilweise abtrennen. Dieser Wasserstoff ist geeignet, dem aus dem Reformierreaktor kommenden rohen Synthesegas zum Einstellen der Stöchiometriezahl zugemischt zu werden.

Für die kostengünstige Verfahrensweise zur Erzeugung des Methanolsynthesegases ist es wichtig, daß aus einer anderen Anlage ein wasserstoffreiches Gas zur Verfügung steht, das man in den Reformierreaktor leiten kann. Beispielsweise fallen in technischen Anlagen zur Dehydrierung solche wasserstoffhaltigen Gase an, die in dieser Weise verwendbar sind. Ein besonders günstiges Beispiel einer anderen Anlage, aus der ein wasserstoffreiches Gas abgezogen werden kann, ist eine solche zur Herstellung von Methyl-Tertiär-Butylether (MTBE). Hierbei geht man üblicherweise von Butan aus, das man zu Isobutan isomerisiert, das Isobutan wird durch Dehydrierung zu Isobutylen umgewandelt und Isobutylen setzt man mit Methanol zu MTBE um. Das bei der Dehydrierung entstehende wasserstoffreiche Abgas wird mindestens teilweise in den Reformierreaktor eingeleitet. Hieraus ergibt sich eine besonders kostengünstige Verfahrenskopplung, weil man einerseits das wasserstoffreiche Abgas der Dehydrierung verwenden kann und weil andererseits Methanol aus der Methanolsynthese zum Umsetzen des Isobutylens zu MTBE zur Verfügung steht.

Für das Verfahren der Erfindung verwendet man ein methanhaltiges Kohlenwasserstoffgas, z.B. Erdgas oder Erdölbegleitgas. Ganz allgemein eignen sich Gasgemische mit C₁- bis C₅-Kohlenwasserstoffen. Das Kohlenwasserstoffgas wird in bekannter Weise vor dem Reformierreaktor entschwefelt, z.B. durch Leiten über Zinkoxid.

Dem Reformierreaktor wird so viel Sauerstoff zugeführt, daß durch partielle Oxidation die gewünschte Temperatur am Austritt des Reaktors erreicht wird. Diese Austrittstemperaturen liegen üblicherweise im Bereich von 800 bis 1300°C und vorzugsweise 850 bis 1100°C. Die Temperatur des in den Reformierreaktor eintretenden Gasgemisches beträgt vorzugsweise 300 bis 650°C.

In an sich bekannter Weise wird dem Reformierreaktor auch Wasserdampf zugeführt, um die Rußbildung zu unterdrücken. Angestrebt wird, daß die sich am Ende des Reaktors einstellende Temperatur mehr als 100°C über der Grenztemperatur für die Rußbildung nach der Boudoir-Reaktion (2CO=C+CO₂) liegt. Vorteilhafterweise kommt man beim Verfahren der Erfindung mit wenig Wasserdampf aus.

Der dem Reformierreaktor zugeführte freie Wasserstoff beschleunigt zusammen mit dem Sauerstoff das Zünden im oberen Bereich des Reformierreaktors, so daß dort die Temperaturen in erwünschter Weise schnell ansteigen und ein zusätzlicher Zündkatalysator oder ein Hilfszündbrenner entbehrlich wird. Dadurch kann man im Bereich des Eintritts von Wasserstoff und Sauerstoff in den Reformierreaktor einen katalysatorfreien Raum vorsehen, der 10 bis 30 % des gesamten Katalysatorvolumens entspricht.

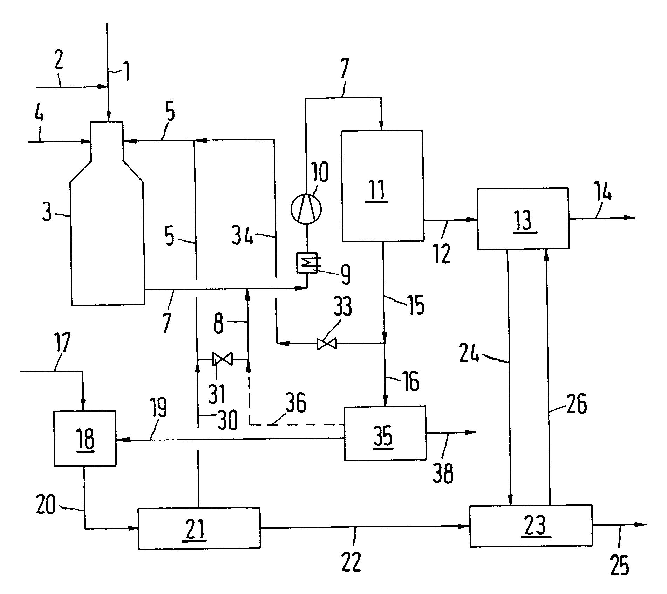

Weitere Einzelheiten der Erfindung werden mit Hilfe der Zeichnung erläutert.

Methanhaltiges Kohlenwasserstoffgas, z.B. Erdgas, wird in der Leitung (1) herangeführt, mit Wasserdampf aus der Leitung (2) gemischt und in einen Reformierreaktor (3) geleitet. Der Reaktor (3) enthält eine Schüttung eines körnigen Nickelkatalysators. Sauerstoff, z.B. aus einer nicht dargestellten Luftzerlegungsanlage, wird dem Reaktor (3) durch die Leitung (4) zugeführt und ein wasserstoffreiches Gas kommt aus der Leitung (5). Im Reaktor (3) herrscht ein Druck von 10 bis 100 bar und vorzugsweise 20 bis 50 bar. Als Produkt der katalytisch autothermen Umsetzung im Reformierreaktor (3) wird durch die Leitung (7) ein rohes Synthesegas abgezogen, das eine Temperatur von 800 bis 1300°C und vorzugsweise 850 bis 1100°C aufweist und höchstens 3 Mol-% Methan enthält. Die Konzentrationen der Hauptkomponenten Wasserstoff, Kohlenmonoxid und Kohlendioxid ergeben eine Stöchiometriezahl von höchstens 2,2.

Falls die Stöchiometriezahl des Synthesegases der Leitung (7) noch nicht im gewünschten Bereich von 1,97 bis 2,2 liegt und angehoben werden muß, erfolgt dies durch Zumischen eines wasserstoffreichen Gases, das aus der Leitung (8) kommt. Das fertige Synthesegas wird, ohne daß eine Behandlung zum Entfernen von CO₂ notwendig ist, nach Abkühlung in einer Kühleinrichtung (9) auf etwa Umgebungstemperatur durch den Verdichter (10) zur Methanolsynthese (11) geführt, die in an sich bekannter Weise ausgestaltet ist. Einen methanolreichen Produktstrom zieht man in der Leitung (12) ab und führt ihn zur Reinigung durch eine Destillation (13), aus der man das fertige Methanol in der Leitung (14) abführt.

Zur Erzeugung des wasserstoffreichen Gases, das in den Leitungen (5) und (8) zur Verfügung steht, gibt es zahlreiche Varianten, von denen hier nur einige dargestellt werden können. Butan kommt aus der Leitung (17) und wird in einer Isomerisierungsanlage (18) mit Wasserstoff aus der Leitung (19) zu Isobutan umgesetzt, das man in der Leitung (20) einer Dehydrierung (21) aufgibt. In der Dehydrierung wird Isobutylen erzeugt, das man in der Leitung (22) zu einer Umsetzung (23) mit Methanol aus der Leitung (24) zuführt. Man erzeugt dadurch MTBE, das in der Leitung (25) abgeführt wird. Wasserreiches Methanol, das in der Umsetzung (23) entsteht, wird durch die Leitung (26) der Destillation (13) aufgegeben.

In der Dehydrierung (21) entsteht ein wasserstoffreiches Abgas, das man in der Leitung (30) abführt und ganz oder teilweise durch die Leitung (5) in den Reformierreaktor (3) leitet. Einen Teil dieses Abgases kann man durch das Ventil (31) und die Leitung (8) dem rohen Synthesegas der Leitung (7) zumischen.

Aus der Methanolsynthese (11) muß ein Restgas in der Leitung (15) entfernt werden, das man auf verschiedene Weise verwenden kann. Eine Möglichkeit ist, es durch das Ventil (33) und die Leitung (34) dem Gas der Leitung (5) zuzumischen und es in den Reformierreaktor (3) zu leiten. Man kann es aber auch ganz oder teilweise durch die Leitung (16) einer Trennanlage (35) aufgeben, in der man ein wasserstoffreiches Gas gewinnt. Die Trennanlage (35) kann z.B. nach dem Prinzip der Druckwechseladsorption arbeiten. Das dabei gewonnene H₂-reiche Gas kann man einerseits in der Leitung (19) abziehen oder aber auch ganz oder teilweise durch die gestrichelte Leitung (36) führen und dem Gas der Leitung (8) zumischen. Ein methanhaltiges Gas wird in der Leitung (38) abgeführt.

Die Umwandlung von Butan zu MTBE ist an sich bekannt, Einzelheiten sind im deutschen Patent 753 753 und in Ullmanns Encyklopädie der technischen Chemie, 3. Auflage, Band 10, Seiten 114-120 beschrieben.

Die nachfolgenden Beispiele, die teilweise berechnet sind, beziehen sich auf 1 Mol CH₄ als Kohlenwasserstoffgas.

### Beispiel 1

Pro Mol CH₄ werden 0,28 Mol H₂, 1,6 Mol H₂O, 0,556 Mol O₂ und 0,0028 Mol N₂ in den Reformierreaktor (3) geleitet, der einen nickelhaltigen Katalysator enthält. Aus der autothermen Umsetzung bei einem Druck von 65 bar und einer Austrittstemperatur von 995°C erhält man ein rohes Synthesegas mit folgender Zusammensetzung:

| | |
|---|---|
| CO₂ | 8,86 Mol-% |
| CO | 20,27 Mol-% |
| H₂ | 67,81 Mol-% |
| CH₄ | 2,97 Mol-% |
| N₂ | 0,09 Mol-% |

Die Stöchiometriezahl dieses Synthesegases beträgt 2,024, d.h., daß das Synthesegas für die Methanolsynthese optimal geeignet ist.

### Beispiel 2

Der katalytisch autothermen Umsetzung wie in Beispiel 1 werden nunmehr pro Mol CH₄ 1,65 Mol H₂O, 0,686 Mol O₂ und 0,0034 Mol N₂ zugeführt, dazu 0,75 Mol eines Abgases aus einer Dehydrierung. Dieses Abgas hat folgende Zusammensetzung:

| | |
|---|---|
| CO₂ | 2,15 Mol-% |
| CO | 0,95 Mol-% |
| H₂ | 67,90 Mol-% |
| CH₄ | 7,15 Mol-% |
| C₂₊ | 9,05 Mol-% |
| N₂ | 12,80 Mol-% |

Die Umsetzung im Reaktor (3) findet bei 65 bar statt, die Austrittstemperatur beträgt 985°C. Das aus dem Reformierreaktor (3) kommende rohe Synthesegas hat folgende Zusammensetzung:

| | |
|---|---|
| CO₂ | 7,68 Mol-% |
| CO | 20,68 Mol-% |
| H₂ | 65,80 Mol-% |
| CH₄ | 3,36 Mol-% |
| N₂ | 2,48 Mol-% |

Die Stöchiometriezahl beträgt 2,049. Das Synthesegas ist also direkt und ohne weitere Behandlung für die Methanolsynthese verwendbar.

Würde man auf das Abgas aus der Dehydrierung verzichten, so müßte man den H₂O-Zusatz auf 2,5 Mol erhöhen, um der Rußbildung im Reformierreaktor vorzubeugen. Dadurch würde aber die Stöchiometriezahl des rohen Synthesegases auf den zu niedrigen Wert von 1,775 absinken.

## Patentansprüche

1. Verfahren zur Erzeugung eines Synthesegases für die Methanolsynthese und Umsetzen des Synthesegases an einem Katalysator zu einem methanolreichen Produktstrom, wobei man ein methanhaltiges Kohlenwasserstoffgas mit Sauerstoff und Wasserdampf in einem Reformierreaktor katalytisch autotherm bei Temperaturen am Reaktoraustritt von 800 bis 1300°C und einem Druck von 10 bis 100 bar umsetzt und aus dem Reformierreaktor ein rohes Synthesegas abzieht, dessen Komponenten vor allem Wasserstoff, Kohlenmonoxid und Kohlendioxid sind, dadurch gekennzeichnet, daß man in den Reformierreaktor zur katalytisch autothermen Umsetzung neben dem Kohlenwasserstoffgas, Sauerstoff und Wasserdampf zusätzlich ein an freiem Wasserstoff reiches Gas aus einer anderen Anlage eingeleitet, wobei man dem Reformierreaktor pro C-Atom 1,2 bis 2,0 Molekühle H₂O, 0,4 bis 0,8 Molekühle O₂ und 0,2 bis 0,5 Molekühle H₂ zuführt, daß man aus dem Reformierreaktor ein rohes Synthesegas mit einem Methangehalt von höchstens 3 Mol.-% abzieht und daraus mit oder ohne Zugabe von Wasserstoff und ohne Entfernung von Kohlendioxid ein für die Methanolsynthese geeignetes Synthesegas erzeugt, in welchem die Konzentrationen der Komponenten H₂, CO und CO₂ ein als Stöchiometriezahl bezeichnetes molares Verhältnis (H₂-CO₂):(CO+CO₂) von 1,97 bis 2,2 ergeben.

2. Verfahren nach Anspruch 1, dadurch gekennzeichnet, daß das aus dem Reformierreaktor abgezogene rohe Synthesegas eine Stöchiometriezahl von 1,97 bis 2,2 aufweist und ohne weitere Behandlung der Methanolsynthese zugeführt wird.

3. Verfahren nach Anspruch 1 oder 2, dadurch gekennzeichnet, daß man aus der Methanolsynthese ein freien Wasserstoff und Kohlenoxide enthaltendes Restgas abzieht und es zusammen mit dem an freiem Wasserstoff reichen, aus einer anderen Anlage kommenden Gas mindestens teilweise in den Reformierreaktor leitet.

4. Verfahren nach Anspruch 1, dadurch gekennzeichnet, daß man aus der Methanolsynthese ein freien Wasserstoff und Kohlenoxide enthaltendes Restgas abzieht, aus dem Restgas freien Wasserstoff teilweise abtrennt und den freien Wasserstoff dem aus dem Reformierreaktor kommenden rohen Synthesegas zum Einstellen der Stöchiometriezahl zumischt.

5. Verfahren nach Anspruch 1, dadurch gekennzeichnet, daß man ein freien Wasserstoff enthaltendes Abgas aus einer Dehydrierung in den Reformierreaktor leitet.

6. Verfahren nach Anspruch 5, dadurch gekennzeichnet, daß man Butan zu Isobutan isomerisiert, das Isobutan durch Dehydrierung zu Isobutylen umwandelt, Isobutylen mit Methanol zu Methyl-Tertiär-Butylether (MTBE) umsetzt und das bei der Dehydrierung entstehende wasserstoffreiche Abgas mindestens teilweise in den Reformierreaktor leitet.

## Claims

1. A method for producing a synthesis gas for methanol synthesis and reaction of the synthesis gas on a catalyst to form a methanol-rich product stream, in which a methane-containing hydrocarbon gas is reacted catalytically and autothermically with oxygen and water vapour in a reforming reactor at temperatures at the reactor exit of 800 to 1300°C and a pressure of 10 to 100 bar, and a crude synthesis gas is withdrawn from the reforming reactor, the constituents of which gas are in particular hydrogen, carbon monoxide and carbon dioxide, characterised in that a gas rich in free hydrogen is additionally introduced from another plant into the reforming reactor for the catalytic autothermic reaction in addition to the hydrocarbon gas, oxygen and water vapour, with 1.2 to 2.0 molecules H₂O, 0.4 to 0.8 molecules O₂ and 0.2 to 0.5 molecules H₂ being supplied to the reforming reactor per C atom, that a crude synthesis gas having a methane content of at most 3 mole percent is withdrawn from the reforming reactor and a synthesis gas suitable for methanol synthesis is produced therefrom with or without the addition of hydrogen and without the removal of carbon dioxide, in which gas the concentration of the constituents H₂, CO and CO₂ yield a molar ratio (H₂-CO₂):(CO+CO₂), called the stoichiometric number, of 1.97 to 2.2.

2. A method according to Claim 1, characterised in that the crude synthesis gas withdrawn from the reforming reactor has a stoichiometric number of 1.97 to 2.2 and is fed to the methanol synthesis without further treatment.

3. A method according to Claim 1 or 2, characterised in that a residual gas containing free hydrogen and carbon oxides is withdrawn from the methanol synthesis and is passed at least partly into the reforming reactor together with the gas which is rich in free hydrogen coming from another plant.

4. A method according to Claim 1, characterised in that a residual gas containing free hydrogen and carbon oxides is withdrawn from the methanol synthesis, free hydrogen is partly separated off from the residual gas and the free hydrogen is admixed to the crude synthesis gas coming from the reforming reactor to set the stoichiometric number.

5. A method according to Claim 1, characterised in that an exhaust gas containing free hydrogen is passed from a dehydrogenation process into the reforming reactor.

6. A method according to Claim 5, characterised in that butane is isomerised to isobutane, the isobutane is converted to isobutylene by dehydrogenation, isobutylene is reacted with methanol to form methyl tert. butyl ether (MTBE) and the hydrogen-rich exhaust gas produced upon the dehydrogenation is passed at least partly into the reforming reactor.

## Revendications

1. Procédé de production de gaz de synthèse pour la synthèse du méthanol et la transformation du gaz de synthèse sur un catalyseur en un courant de produit riche en méthanol qui consiste à faire réagir catalytiquement et de manière autotherme, à des températures à la sortie du réacteur de 800 à 1300°C sous une pression de 10 à 100 bar, un hydrocarbure gazeux contenant du méthane sur de l'oxygène et de la vapeur d'eau et à soutirer du réacteur de reformage un gaz de synthèse brut dont les constituants sont surtout de l'hydrogène, du monoxyde de carbone et du dioxyde de carbone, caractérisé en ce qu'il consiste à envoyer dans un réacteur de reformage pour la transformation catalytique et autotherme, outre l'hydrocarbure gazeux, l'oxygène et la vapeur d'eau un gaz riche en hydrogène libre provenant d'une autre installation, en envoyant au réacteur de reformage, par atome de carbone de 1,2 à 2,0 molécule H₂0, de 0,4 à 0,8 molécule d'0₂ et de 0,2 à 0,5 molécule d'H₂, à soutirer du réacteur de reformage un gaz de synthèse brut ayant une teneur en méthane de 3% en mole au plus et à produire à partir de celui-ci, avec ou sans addition d'hydrogène et sans élimination de dioxyde de carbone, un gaz de synthèse qui convient à la synthèse du méthanol et dans lequel les concentrations des constituants que sont H₂, CO et CO₂ donnent un rapport molaire, désigné par indice stoechiométrique (H₂-CO₂) : (CO+CO₂), de 1,97 à 2,2.

2. Procédé suivant la revendication 1, caractérisé en ce que le gaz de synthèse brut soutiré du réacteur de reformage a un indice stoechiométrique de 1,97 à 2,2 et est envoyé sans autre traitement à la synthèse du méthanol.

3. Procédé suivant la revendication 1 ou 2, caractérisé en ce qu'il consiste à soutirer de la synthèse du méthanol un gaz résiduel contenant de l'hydrogène libre et des oxydes de carbone et à l'envoyer au moins partiellement au réacteur de reformage en même temps que le gaz riche en hydrogène libre et provenant d'une autre installation.

4. Procédé suivant la revendication 1 caractérisé en ce qu'il consiste à soutirer de la synthèse du méthanol un gaz résiduel contenant l'hydrogène libre et des oxydes de carbone, à séparer en partie de l'hydrogène libre du gaz résiduel et à mélanger l'hydrogène libre au gaz de synthèse brut venant du réacteur de reformage pour régler l'indice stoechiométrique.

5. Procédé suivant la revendication 1 caractérisé en ce qu'il consiste à envoyer un effluent gazeux contenant l'hydrogène libre et provenant d'une déshydrogénation au réacteur de reformage.

6. Procédé suivant la revendication 5, caractérisé en ce qu'il consiste à isomériser du butane en isobutane, à transformer l'isobutane en isobutylène par déshydrogénation, à faire réagir de l'isobutylène sur du méthanol pour obtenir de l'oxyde méthylbutylique tertiaire (MTBE), et à envoyer l'effluent gazeux riche en hydrogène se formant lors de la déshydrogénation au moins en partie au réacteur de reformage.
